(19) **Europäisches Patentamt / European Patent Office / Office européen des brevets**

(11) **EP 3 631 423 B1**

(12) **FASCICULE DE BREVET EUROPEEN**

(45) Date de publication et mention
de la délivrance du brevet:
**14.08.2024   Bulletin 2024/33**

(21) Numéro de dépôt: **18727711.6**

(22) Date de dépôt: **15.05.2018**

(51) Classification Internationale des Brevets (IPC):
**G01N 21/77** *(2006.01)*      **G01N 31/22** *(2006.01)*
**G01N 33/497** *(2006.01)*

(52) Classification Coopérative des Brevets (CPC):
**G01N 31/22; G01N 21/77; G01N 33/4972;**
G01N 2021/7773

(86) Numéro de dépôt international:
**PCT/EP2018/062605**

(87) Numéro de publication internationale:
**WO 2018/215247 (29.11.2018 Gazette 2018/48)**

(54) **DISPOSITIF OPTIQUE DE DÉTECTION ET DE QUANTIFICATION DE COMPOSÉS VOLATILS**

OPTISCHE VORRICHTUNG ZUR ERFASSUNG UND QUANTIFIZIERUNG VON FLÜCHTIGEN
VERBINDUNGEN

OPTICAL DEVICE FOR DETECTING AND QUANTIFYING VOLATILE COMPOUNDS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **22.05.2017   FR 1754533**

(43) Date de publication de la demande:
**08.04.2020   Bulletin 2020/15**

(73) Titulaires:
• **Université d'Aix Marseille
13007 Marseille 7 (FR)**
• **Centre National de la Recherche Scientifique
75016 Paris (FR)**

(72) Inventeurs:
• **ALVAREZ, Elsa
13008 Marseille (FR)**
• **GROSSO, David
13190 Allauch (FR)**
• **ABBARCHI, Marco
13009 Marseille (FR)**

(74) Mandataire: **INNOV-GROUP
310, avenue Berthelot
69372 Lyon Cedex 08 (FR)**

(56) Documents cités:
**WO-A1-2011/058308      WO-A1-2014/078512
WO-A2-2005/005959      WO-A2-2010/075014**

**Description**

La présente invention concerne un dispositif optique de détection et de quantification de composés volatils tels que l'alcool et la détermination de l'alcool sous forme du taux alcoolémie.

La consommation d'alcool par des automobilistes est un enjeu de société majeur. En 2015 en France, l'alcool était en cause dans près d'un tiers des accidents mortels et constitue de fait l'une des premières causes de mortalité sur les routes. Ce chiffre représente plus de 1000 personnes tuées chaque année. De nuit, la consommation excessive d'alcool est impliquée dans la moitié des accidents mortels.

C'est pourquoi la consommation d'alcool est interdite dans certains états ou pour le moins strictement limitée, notamment envers les jeunes conducteurs.

Pour contrôler le taux d'alcoolémie d'un usager de la route, on connaît de nombreux dispositifs de mesure et de contrôle, en particulier des éthylotests. Ces dispositifs mesurent le taux d'éthanol contenu dans l'haleine expirée par une personne, ce qui permet de déterminer dans une certaine plage son taux d'alcoolémie.

Parmi ces éthylotests, on distingue en particulier des éthylotests chimiques et les éthylotests électroniques.

Pour les éthylotests chimiques, ceux-ci fonctionnent selon le principe d'oxydoréduction qui s'effectue entre le dichromate de potassium, l'acide sulfurique et l'éthanol. Il s'agit donc d'une application concrète de la chimie organique. En effet, on observe une coloration du coton en vert/bleu à la fin de la réaction. Ainsi, l'éthanol et le dichromate de potassium ont réagi ensemble : le test est positif. Ces tests sont peu onéreux en tant que tels mais à usage unique. De plus, ces tests n'ont qu'une durée limitée d'utilisation. Enfin, ces tests donnent plutôt une indication du taux d'alcoolémie et ont une faible précision de mesure.

Les éthylotests électroniques permettent de résoudre un certain nombre de ces inconvénients.

L'éthylotest électronique analyse l'air alvéolaire expiré grâce à un détecteur semi-conducteur réalisé sous forme d'un capteur électrochimique sensible à l'éthanol. Dans le cas de l'éthylotest électronique, un courant se crée lorsque la présence de molécules d'éthanol dans l'haleine est relativement importante. Aussi, l'intensité du courant est liée à la quantité d'éthanol dans l'air expiré. Le semi-conducteur permet alors le passage du courant généré jusqu'à un microprocesseur qui l'évalue et le convertit en une mesure lisible sur un afficheur.

Les éthylotests électroniques sont fiables et précis. Ils peuvent être utilisés de façon répétée avec différents usagers de la route, seule une petite pièce plastique buccale destinée à être en contact avec la bouche de l'usager est jetable et à usage unique.

Cependant, ces éthylotests électroniques sont très complexes et onéreux pour atteindre la précision requise. De plus, leur temps de réponse est assez faible typiquement entre 3s et 5s.

La présente invention a pour objet de proposer une alternative réutilisable et moins onéreuse aux éthylotests électroniques à semi-conducteur et qui présente une bonne fiabilité de mesure.

A cet effet, la présente invention propose un dispositif optique de détection et de quantification de composés volatils, comportant :

- un élément réflecteur sensible dont le taux de réflexion varie en fonction du taux d'éthanol contenu dans une atmosphère à tester, l'élément réflecteur sensible comportant:

   ◦ un substrat,

   ◦ une couche sensible comprenant de la silice sol-gel à caractère hydrophobe microporeuse présentant une épaisseur supérieure à 250 nm, notamment comprise entre 400 nm et 1200nm, une taille moyenne des pores inférieure à 2nm et une porosité inférieure à 25%, la couche sensible étant destinée à être mise en présence avec une atmosphère à tester, notamment chargée ou non d'éthanol, et plus particulièrement de l'haleine expirée d'une personne,

- une source de lumière monochromatique ou quasi-monochromatique disposée pour éclairer la couche sensible sous un angle incident,
- un détecteur de lumière pour mesurer l'intensité réfléchie par l'élément réflecteur sous un angle de détection, et
- une unité de traitement et de calcul configurée pour déduire de l'intensité réfléchie par l'élément réflecteur un paramètre correspondant à un taux d'alcoolémie.

Le dispositif de la présente invention est défini dans la revendication 1.

Grâce à l'invention, on dispose d'un dispositif optique de détection et de quantification de composés volatils tels que l'alcool et notamment d'un dispositif de détermination du taux d'alcoolémie qui est efficace et très sensible avec un excellent temps de réponse pour détecter le taux d'alcoolémie d'une personne.

Le dispositif selon l'invention peut en outre présenter une ou plusieurs des caractéristiques suivantes prises seules ou en combinaison :
La couche sensible peut ne pas comprendre d'agents structurants, notamment de CTAB, de DTAB ou de F127.

Le substrat possède selon un exemple un indice de réfraction supérieur à 1,8, de préférence supérieur à 2.5, notamment supérieur à 3 pour une longueur d'onde comprise entre 250nm et 1500nm.

Le substrat est par exemple réalisé en un matériau semi-conducteur, notamment en silicium.

L'angle incident et l'angle de détection sont respectivement notamment compris entre 30° et 75°.

La longueur d'onde de la source de lumière est monochromatique et choisie avec l'angle d'incidence de façon à coïncider avec la position en longueur d'onde d'une inflexion ($I_1$, $I_2$, $I_3$) entre deux pics d'interférence constructive et destructive du spectre de réflexion de l'élément réflecteur.

La longueur d'onde de la source de lumière est comprise entre 500nm et 1000nm.

La couche sensible présente un indice de réfraction compris entre 1,2 et 1,6, plus particulièrement compris entre 1,3 et 1,4 pour une longueur d'onde comprise entre 500nm et 1000nm.

Le dispositif de détection peut comporter en outre

- un élément réflecteur sensible additionnel dont le taux de réflexion varie en fonction du taux d'humidité contenu dans une atmosphère à tester,

  - un détecteur de lumière additionnel pour mesurer l'intensité réfléchie par l'élément réflecteur sensible additionnel sous un angle de détection,

  - l'unité de traitement et de calcul étant configurée pour déduire des intensités réfléchies d'une part par l'élément réflecteur et d'autre part par l'élément réflecteur sensible additionnel un paramètre correspondant à un taux d'alcoolémie en tenant compte de l'influence de l'humidité dans l'atmosphère à tester.

Le procédé de fabrication d'un élément réflecteur sensible pour un dispositif optique de détection et de quantification de composés volatils tel que défini ci-dessus, peut être caractérisé en ce que

- on réalise une solution sol-gel en dissolvant du TEOS et du MTEOS dans une solution composée de PrOH, d'acide chlorhydrique et d'eau,

- on dépose la solution sol-gel sur le substrat sous une humidité relative comprise entre 40% et 90%, notamment entre 50% et 60% pour obtenir une couche sensible,

- on soumet la couche sensible à une étape de calcination à une température comprise entre 250°C et 450°C, notamment à 350°C, ceci pendant une durée supérieure à 5 min, notamment pendant 10 min.

Le procédé peut présenter une ou plusieurs des caractéristiques suivantes prises seules ou en combinaison :

L'épaisseur de la couche sensible est par exemple supérieure à 250 nm, notamment comprise entre 400 nm et 1200nm.

L'élément réflecteur sensible pour un dispositif optique de détection et de quantification de composés volatils tel que défini ci-dessus, peut être caractérisé en ce que l'élément réflecteur comprend

  - un substrat, et

  - une couche sensible comprenant de la silice sol-gel hydrophobe microporeuse présentant une épaisseur supérieure à 250 nm, notamment comprise entre 400 nm et 1200nm, une taille moyenne des pores inférieure à 2nm et une porosité inférieure à 25%, la couche sensible étant destinée à être mise en présence avec une atmosphère à tester, notamment chargée ou non d'éthanol, et plus particulièrement de l'haleine expirée d'une personne.

L'élément réflecteur sensible présente un taux de réflexion variant en fonction du taux d'éthanol contenu dans une atmosphère à tester.

L'invention concerne en outre un procédé de fabrication d'un dispositif de détection optique de détection et de quantification de composés volatils tel que défini ci-dessus, caractérisé en ce que

- on dépose une couche sensible sur un substrat pour former un élément réflecteur sensible,
- on détermine le spectre de réflexion de l'élément réflecteur sensible,
- la longueur d'onde de la source de lumière est monochromatique, ou quasi monochromatique, et choisie avec l'angle d'incidence du rayon lumineux sur l'élément réflecteur de façon à coïncider avec la position en longueur d'onde d'une inflexion du spectre de réflexion de l'élément réflecteur.

Le procédé de fabrication de la présente invention est défini dans la revendication 10.

Selon un aspect de ce procédé, le substrat possède par exemple un indice de réfraction compris entre 1.8 et 4, notamment entre 2.5 et 3.5, la couche sensible possède une épaisseur supérieure à 250 nm, notamment comprise entre 400 nm et 1200nm, et présent un indice de réfraction compris entre 1.2 et 1.6, plus particulièrement entre 1.3 et 1.4 et la longueur d'onde de la source de lumière est comprise entre 500nm et 1000nm.

Selon un autre aspect, l'élément réflecteur est réalisé selon le procédé tel que défini ci-dessus.

L'invention concerne aussi un procédé de détection optique de détection et de quantification de composés volatils dans une atmosphère à tester, caractérisé en ce qu'il comprend les étapes suivantes :

- on mesure l'intensité réfléchie par un élément réflecteur sensible tel que défini ci-dessus dont le taux de réflexion varie en fonction du taux d'éthanol contenu dans l'atmosphère à tester,

- on mesure l'intensité réfléchie par un élément réflecteur sensible additionnel tel que défini précédemment dont le taux de réflexion varie en fonction du taux d'humidité contenu dans l'atmosphère à tester,

- dans une zone temporelle prédéterminée d'intérêt pour laquelle la dérivée dans le temps est la même pour les deux intensités mesurées en cas d'absence d'éthanol, on détermine la différence de la dérivée temporelle des deux intensités mesurées pour réaliser une corrélation avec une courbe de calibrage afin de quantifier l'éthanol contenu dans l'atmosphère à tester.

La longueur d'onde de la source de lumière est choisie de façon à coïncider avec la position en longueur d'onde d'une inflexion entre deux pics d'interférence constructive et destructive du spectre de réflexion de l'élément réflecteur sensible.

D'autres avantages et caractéristiques apparaîtront à la lecture de la description de l'invention, ainsi que des figures suivantes sur lesquelles :

- la figure 1 montre un schéma simplifié d'un dispositif de détection et de quantification de composés volatils selon l'invention, notamment pour la détermination d'un taux alcoolémie,

- la figure 2A montre un schéma de principe agrandi de la partie détection du dispositif de la figure 1,

- la figure 2B montre un schéma de principe pour illustrer les équations de Fresnel,

- la figure 3 représente un graphe montrant un paramètre de mesure représentant l'intensité du signal réfléchi de lumière en fonction de la concentration d'éthanol dans l'air humide,

- la figure 4 représente un graphe montrant pour deux épaisseurs différentes la réflectivité en fonction de la longueur d'onde, et

- la figure 5 montre sur un graphe la variation de l'intensité réfléchie en fonction de la longueur d'onde pour une couche d'une épaisseur donnée lorsque cette dernière subit une variation d'indice de réfraction d'une valeur prédéfinie.

- la figure 6 montre de façon schématique un autre mode de réalisation du dispositif de détection et de quantification de composés volatils selon l'invention, notamment pour la détermination d'un taux alcoolémie,

- la figure 7 montre sur un graphe des paramètres de mesure en fonction du temps,

- la figure 8 montre sur un graphe en fonction du temps un traitement des paramètres de mesure de la figure 7,

- la figure 9 montre une courbe de corrélation entre une teneur en éthanol d'une part et des valeurs de mesures traitées selon la figure 8 d'autre part.

Sur toutes les figures, les éléments identiques portent les mêmes numéros de référence.

Les réalisations suivantes sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées et/ou interchangées pour fournir d'autres réalisations.

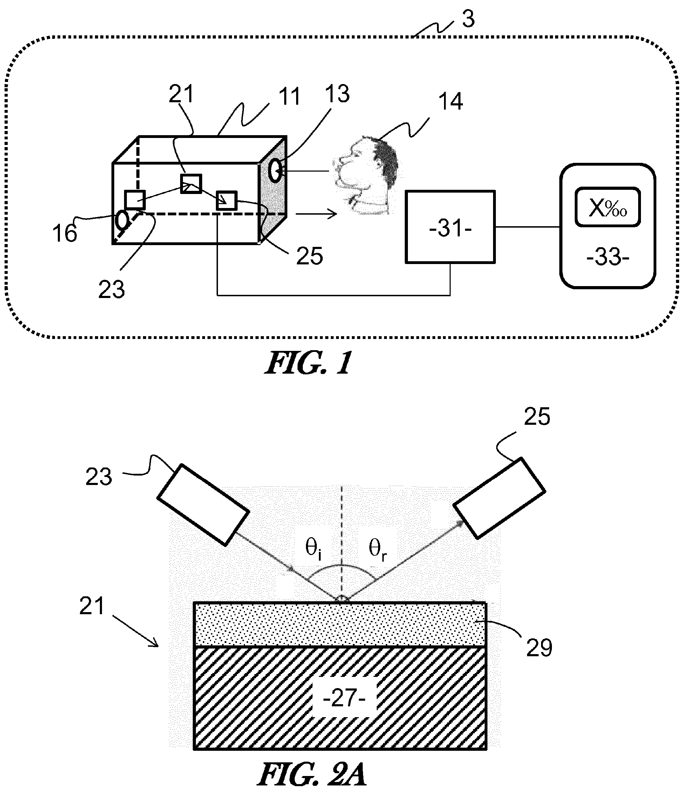

La figure 1 montre de façon schématique une vue de côté d'un dispositif de détection optique 3 de détection et de quantification de composés volatils, notamment en vue de la détermination d'un taux alcoolémie.

Ce dispositif optique 3 comprend une enceinte de mesure 11 avec une entrée 13 destinée à recevoir un flux d'atmosphère à tester, notamment un flux alvéolaire d'une personne 14 exhalant son haleine.

Cette enceinte de mesure 11 peut par exemple être réalisée en matière plastique et ses dimensions sont par exemple choisies de manière à contenir un volume un peu plus faible que le volume moyen expiré par une personne 14, de sorte que lors d'un contrôle d'alcoolémie, la personne 14 souffle dans l'enceinte 11 et l'air contenu dans l'enceinte 11 est complètement remplacé par l'air alvéolaire de la personne 14 expirant.

Pour des raisons d'hygiène, on prévoit pour l'entrée une pièce buccale à usage unique (non représentée) qui se fixe par exemple par clipsage sur l'entrée 13 de l'enceinte 11.

La paroi de fond de l'enceinte 11 opposée à celle présentant l'entrée 13 comprend une sortie 16 de l'air alvéolaire.

Sur la figure 1, l'enceinte 11 possède une forme simple parallélépipédique, mais d'autres formes sont envisageables, notamment afin de pouvoir bien mélanger le flux entrant par l'entrée 13. Ainsi, l'enceinte 11 peut comporter des déflecteurs (non représentés) permettant de créer un flux tourbillonnaire pour bien mélanger le flux entrant. Le même effet peut être obtenu par exemple par une forme particulière des parois de l'enceinte 11, par exemple en forme de volute.

Selon un développement, à la sortie 16, on peut installer un pulseur d'air, par exemple mu par un moteur électrique, pour pouvoir purger l'air dans l'enceinte 11. En effet, après une mesure et pour rendre le dispositif 3 le plus rapidement opérationnel à nouveau, il est nécessaire de remplacer l'atmosphère par exemple par de l'air ambiant sans éthanol.

Dans le cas présent, les parois de l'enceinte 11 de mesure sont par exemple réalisées en matière opaque, notamment en noir, par exemple en polycarbonate ou en poly(méthacrylate de méthyle) chargé en noir de carbone pour empêcher que de la lumière ambiante ne puisse pénétrer dans l'enceinte 11 afin de réduire le risque d'une perturbation des mesures par de la lumière ambiante.

On peut par ailleurs prévoir pour les parois internes de l'enceinte 11 un revêtement absorbant de lumière pour diminuer davantage le risque de perturbation des mesures par de la lumière ambiante

A l'intérieur de l'enceinte 11 sont disposés un élément réflecteur 21 sensible dont le taux de réflexion varie en fonction du taux d'éthanol contenu dans une atmosphère à tester, une source de lumière monochromatique 23 disposée pour éclairer l'élément réflecteur sensible 21 sous un angle incident et un détecteur de lumière 25 pour mesurer l'intensité réfléchie par l'élément réflecteur sensible 21.

Par exemple la source de lumière monochromatique 23 et le détecteur de lumière 25 sont disposés sur une paroi de l'enceinte 11 tandis que l'élément réflecteur 21 sensible est disposé sur la paroi opposée de l'enceinte 11.

Bien entendu, d'autres montages sont possibles pour autant que le détecteur de lumière 25 puisse détecter la lumière réfléchie par l'élément réflecteur 21.

La source de lumière monochromatique 23 est par exemple un laser, en particulier une diode laser ou une LED. La longueur d'onde est par exemple comprise entre 250nm et 1200 nm environ. La source de lumière 23 peut émettre un flux monochromatique de lumière de manière continue ou pulsée.

Le détecteur de lumière 25 peut être tout appareil de prise de vue apte à mesurer l'intensité réfléchie par l'élément réflecteur 21 sensible, comme par exemple une caméra. Selon un mode de réalisation préférentiel le détecteur de lumière 25 est une photodiode au silicium.

L'élément réflecteur 21 sensible possède un taux de réflexion qui varie proportionnellement en fonction du taux d'éthanol contenu dans une atmosphère à tester dans l'enceinte 11 afin de pouvoir mesurer la teneur en éthanol de l'atmosphère composée par le flux alvéolaire de la personne 14 exhalant son haleine de manière à pouvoir déterminer son taux d'alcoolémie grâce à la détection de l'intensité lumineuse réfléchie par cet élément réflecteur 21.

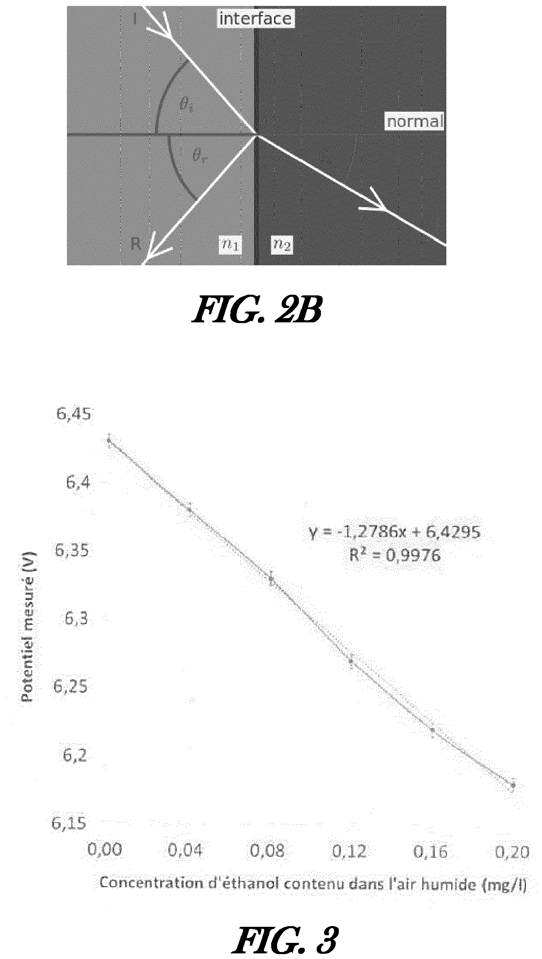

A cet effet, comme montré plus en détail sur la figure 2, l'élément réflecteur sensible 21 comporte un substrat 27 et une couche sensible 29.

Le substrat 27 possède un indice de réfraction, en particulier un indice de réfraction supérieur à 1,8, de préférence supérieur à 2.5, notamment supérieur à 3.4 pour une longueur d'onde comprise entre 400nm et 1500nm.

Selon un mode de réalisation, le substrat 27 est réalisé en un matériau semi-conducteur, notamment du silicium. En alternative, le substrat 27 peut aussi être en verre dont la face arrière, c'est-à-dire celle opposée à la source de lumière 23 et le détecteur de lumière 25, est réfléchissante, par exemple du fait d'un revêtement métallique notamment du type miroir.

Sur ce substrat 27 est déposée une couche sensible 29 en silice sol-gel microporeuse présentant une épaisseur supérieure à 250 nm, notamment comprise entre 400 nm et 1200nm, une taille moyenne des pores inférieure à 2nm et une porosité inférieure à 25%. La couche sensible 29 microporeuse est rendue, au moins en partie, hydrophobe, en particulier, les micropores de la couche sensible 29 accessibles présentent un caractère hydrophobe.

La couche sensible 29 est donc destinée à être mise en présence de l'atmosphère à tester, chargée ou non d'éthanol, et plus particulièrement de l'haleine expirée d'une personne 14, c'est-à-dire l'air alvéolaire saturé en vapeur d'eau afin de permettre une modification éventuelle de l'intensité lumineuse réfléchie par l'élément réflecteur 21 pour déterminer la quantité d'éthanol contenue dans cette atmosphère à tester de manière à obtenir le taux d'alcoolémie de la personne 14.

En effet, après la consommation d'alcool, celui-ci passe dans le sang et se répand dans tout le corps et on peut détecter sa présence et mesurer le taux d'alcoolémie par la mesure de l'éthanol contenu dans l'air alvéolaire expiré par une personne 14.

Les rayons de lumière de la source de lumière 23 sont dirigés dans le présent exemple directement sur la couche sensible 29 de l'élément réflecteur 21 en présence d'une atmosphère à tester, chargée ou non en éthanol et plus particulièrement de l'haleine expirée d'une personne 14. Les rayons sont réfléchis vers un détecteur de lumière 25 et en présence d'éthanol, l'indice de réfraction de l'élément réflecteur 21 varie et permet d'en déduire le taux d'alcoolémie, par la mesure de l'intensité de la lumière réfléchie comme ce sera expliqué un peu plus loin.

Le dispositif de détection 3 comporte de plus une unité 31 de traitement et de calcul reliée à la source de lumière 23 et au détecteur de lumière 25 pour commander leur fonctionnement et configurée pour déduire de l'intensité réfléchie par la couche sensible 29 un paramètre correspondant à un taux d'alcoolémie. L'unité 31 de traitement et de calcul est, par exemple, reliée aussi à un afficheur 33 permettant d'afficher le taux d'alcoolémie mesuré.

Au fur et à mesure que l'air à l'intérieur de l'enceinte 11 est remplacé par l'air alvéolaire de la personne 14, l'éthanol éventuellement contenu dans l'air alvéolaire est adsorbé par la couche sensible 29 tandis que l'adsorption de l'eau est minimisée du fait des propriétés majoritairement hydrophobes de la couche sensible 29.

Cette adsorption réversible de l'éthanol engendre une modification réversible de la constante diélectrique de la couche sensible 29 et donc un changement de l'indice de réfraction de cette dernière. En effet, de manière simplifiée, l'indice de réfraction évolue comme la racine de la constante diélectrique.

Selon un mode de réalisation particulier de l'invention, les inventeurs ont observé qu'en présence d'une atmosphère saturée en humidité, les modifications de l'indice de réflectivité de l'élément réflecteur ont été plus importantes.

Selon un autre mode de réalisation de l'invention, il serait avantageux d'utiliser le dispositif dans un milieu pour lequel le taux d'humidité serait contrôlé.

Selon une approche simplifiée avec les équations de Fresnel (voir figure 2B) ne prenant pas en compte la présence du substrat 27, on peut poser :

$$Rs = \left| \frac{n_1 \cos\left(\theta_i\right) - n_2 \cos\left(\theta_t\right)}{n_1 \cos\left(\theta_i\right) + n_2 \cos\left(\theta_t\right)} \right|^2$$

$$Rp = \left| \frac{n_1 \cos\left(\theta_t\right) - n_2 \cos\left(\theta_i\right)}{n_1 \cos\left(\theta_t\right) + n_2 \cos\left(\theta_i\right)} \right|^2$$

$$R = 1/2\left(Rs + Rp\right)$$

où

- Rs représente la réflectivité pour une polarisation incidente s,
- Rp représente la réflectivité pour une polarisation incidente p,
- R représente la réflectivité dans le cas de lumière non-polarisée.
- $\theta_i$ représente l'angle de l'onde incidente
- $\theta_t$ représente l'angle de l'onde transmise,
- $\theta_r$ est l'angle de détection des rayons de lumière par rapport à la normale de l'élément réflecteur sensible 21,
- $n_1$ est l'indice de réfraction de l'air, $n_1 = 1$ = constante,
- $n_2$ est l'indice de réfraction de la couche sensible 29 qui varie en fonction du taux d'éthanol contenu dans l'atmosphère à tester.

Etant donné que dans cette formule, seul $n_2$ varie en fonction du taux d'éthanol contenu dans l'atmosphère à tester, $n_1$, $\theta_1$, $\theta_2$ restant constants, on peut donc en déduire que le taux de réflexion diminue linéairement avec une augmentation de l'indice de réfraction $n_2$. Il se trouve que l'indice de réfraction $n_2$ varie linéairement avec le taux d'éthanol adsorbé par la couche sensible 29.

Avec les équations de Fresnel complexes tenant compte de l'existence du substrat 27 (ce qui sera discuté plus loin un peu plus en détail), on obtient un résultat similaire, c'est-à-dire que l'indice de réfraction $n_2$ varie linéairement avec le taux d'éthanol adsorbé par la couche sensible 29.

Les angles incidents et de détection $\theta_1$, $\theta_2$ sont par exemple respectivement compris entre 30° et 75°. $\theta_1$ et $\theta_2$ peuvent être égaux (cas de réflexion directe), mais ne le sont pas nécessairement.

La figure 3 est un graphe représentant une mesure montrant une tension de mesure à la sortie du détecteur de lumière 25, ce qui représente un paramètre correspondant à l'intensité réfléchie en fonction de la concentration d'éthanol contenue dans l'air humide.

On constate la forte corrélation linéaire entre les deux grandeurs de sorte qu'avec un simple calibrage, il est possible de quantifier un composé volatil (ici de l'éthanol) dans l'atmosphère à tester, en particulier pour mesurer le taux d'alcoolémie par le dispositif de détection 3.

Comme dans le cas de l'haleine, l'air expiré est saturé en humidité (taux d'humidité égal à 100%), il n'est pas nécessaire de faire une correction des mesures en fonction du taux d'humidité.

Cependant, dans certains cas de dérive, une correction peut être effectuée à l'aide d'un second capteur sensible à l'humidité, ce qui sera expliqué plus loin.

En outre, comme le processus d'adsorption est réversible, le dispositif de détection 3 se prête particulièrement bien à une utilisation répétée, d'autant plus que le temps de réponse du dispositif de détection 3 est inférieur à quelques secondes.

On va par la suite détailler les propriétés et le procédé de fabrication de la couche sensible 29 en silice sol-gel à caractère hydrophobe microporeuse.

Cette couche présente une épaisseur supérieure à 250 nm, notamment comprise entre 400 nm et 1200nm.

La taille moyenne des pores est inférieure à 2nm.

On peut déterminer le diamètre moyen des pores par une méthode connue de volumétrie qui est par exemple décrite en détail dans l'article « Porosity and mechanical properties of mesoporous thin films assessed by environmental ellipsometric porosimetry » Cedric Boissière et al, paru dans American Chemical Society, 2005. Langmuir : the ACSjournal of surfaces and colloids 2005, 21, 12362-71.

Le volume poreux de la couche sensible 29 disposée sur le substrat 27 est inférieur à ou égal à 25%. Il s'agit de la fraction volumique du pore dans le volume de la couche poreuse.

La surface de pores accessibles de la couche sensible 29 disposée sur le substrat 27 est supérieure à 140$cm^2/cm^2$.

La surface de pores accessibles de la couche poreuse est déterminée de la façon suivante :
Calcul de la surface accessible de pore totale S en cm2/cm2 (à ramener à une unité de surface de s = 1 $cm^2$) :

$$S = 4*V*e*s/D$$

Où

-   V est le volume poreux, par exemple inférieur ou égal à 25%,

-   e est l'épaisseur de la couche poreuse hydrophobe 7, par exemple comprise entre 400nm et 1000nm, notamment entre 500nm et 700nm,

-   D est le diamètre moyen des pores, par exemple inférieur ou égal à 2 nm,

-   s surface unitaire de 1$cm^2$

Selon un mode de réalisation, la couche sensible 29 est réalisée sans ajout d'agents structurants c'est à dire sans ajout d'espèces chimiques de nature minérale ou organique autour desquelles le matériau pourrait s'organiser. Des exemples d'agents structurants courants sont notamment le CTAB (bromure d'hexadecyltrimethylammonium), DTAB (bromure de decyltrimethylammonium) ou F127 (le copolymère triblocs Pluronic F127 ([PEO]$_{106}$-[PO]$_{70}$-[PEO]$_{106}$)).

Selon un mode de réalisation préféré de l'invention, la couche sensible 29 est préparée par un procédé sol-gel pour lequel la couche sensible 29 sol-gel comprend au moins un oxyde métallique rendu plus hydrophobe, préférentiellement de la silice rendue plus hydrophobe par l'utilisation de methyltriethoxysilane (MTEOS), idéalement 50 % molaire, et de tetraethylorthosilicate (TEOS) utilisé comme précurseur de silice. D'autres voies de réalisation par post-greffage ou par l'utilisation d'autres agents précurseurs méthylés de silice, peuvent être envisagées et sont bien connues de l'homme du métier.

Cette couche sensible 29 est déposée par exemple sur une seule face du substrat 27.

Selon un mode de réalisation préféré, le solvant utilisé pour la préparation de la couche sensible 29 est le 2-propanol (PrOH), en particulier, le solvant ne comprend pas d'éthanol (EtOH).

La couche sensible 29 est ensuite soumise à un traitement de stabilisation qui a pour objectifs la consolidation du réseau inorganique par condensation des précurseurs MTEOS et TEOS en $SiO_2$ méthylée (ou si un oxyde métallique est utilisé, en unités oxo-métalliques méthylées) et la formation de la porosité par élimination des composés volatils, produits de la condensation, par exemple l'eau produite lors de la condensation des précurseurs MTEOS et TEOS. Ce traitement de stabilisation est généralement un traitement thermique, par exemple une calcination à une température comprise entre 200°C et 400°C, notamment à 350°C. Il en résulte des pores de dimension nanométrique.

Ce traitement de stabilisation peut également être obtenu par un traitement chimique en phase vapeur, par exemple par de la vapeur d'ammoniac, suivi d'une lixiviation.

La couche sensible 29 présente une variation de l'indice de réfraction en fonction de l'éthanol adsorbé. Cette variation peut être de l'ordre de quelques % dans la plage de mesure du taux d'alcoolémie, notamment entre 0-1% environ.

Le substrat 27 est choisi selon sa capacité à résister aux conditions du traitement et présenter une chimie de surface permettant la formation de liaisons chimiques fortes (de types ionocovalentes) avec la couche sensible 29.

Comme déjà indiqué ci-dessus, le substrat 27 peut être en silicium.

Il en résulte des pores de très petite taille et une porosité de l'ordre de 10-20% en volume, mais une variation plus forte de l'indice de réfraction en fonction de l'éthanol adsorbé. Cette variation peut être de l'ordre de quelques % dans la plage de mesure du taux d'alcoolémie, notamment entre 0-1% environ.

Selon un exemple de l'invention, des films microporeux de silice ont été préparés à partir d'une solution comprenant du tetraethoxysilane (TEOS) /MTEOS (methyltriethoxysilane) /HCl /$H_2O$ /PrOH avec des proportions molaires de 0.5/0.5/0.17/4/5.8 respectivement. La taille moyenne des pores est inférieure à 2 nm.

Les films microporeux sont obtenus à partir de 0.5 mol de TEOS et 0.5 mol de MTEOS qui sont tout d'abord dissous dans un mélange composé de 5.8 mol de 2-propanol (PrOH), 0.17 mol d'acide chlorhydrique et de 4 mol d'eau.

Cette solution peut être mise sous agitation pendant par exemple au moins 24 h à température ambiante avant d'être utilisée. Selon une variante, on chauffe à 70° durant environ 30 minutes, puis on laisse reposer quelques heures, en particulier entre 3-5h, à température ambiante.

Les solutions à base de TEOS et de MTEOS peuvent être combinées avec d'autres types de précurseurs d'oxydes métalliques (métal organique, organométallique, sels) d'autres oxydes métalliques, notamment des oxydes de Ti, Al, Zr, Zn, Ca, Mg, et/ou de Fe en toutes proportions.

Puis pour obtenir la couche sensible 29, un film sol-gel de la solution précédemment décrite est déposé sur le substrat 27 par exemple par dépôt par voie liquide (revêtement par centrifugation ou « spin coating » en anglais, par pulvérisation, par impression à la façon jet d'encre, trempage ou « dip coating » en anglais) par exemple sur une seule face du substrat 27.

Le cas du trempage est préféré pour l'homogénéité d'épaisseur.

La vitesse de retrait en cas de trempage permet de contrôler l'épaisseur de la couche sensible 29 et est généralement comprise entre 0,001 et 20 mm/s afin d'ajuster l'épaisseur de la couche à la valeur souhaitée.

Le dépôt de la couche sensible 29 sur le substrat 27 est réalisé sous une humidité relative, de préférence comprise entre 10% et 99%, et plus préférentiellement encore comprise entre 50% et 90 % afin d'obtenir une couche présentant de bonnes propriétés d'adsorption d'éthanol.

L'élément réflecteur 25 sensible comportant le substrat 27 et la couche sensible 29 est ensuite calciné à une température comprise entre 250°C et 450°C, idéalement à 350°C, ceci pendant une durée supérieure à 5 min, notamment pendant 10 min.

Ce traitement thermique permet de stabiliser et de consolider la couche sensible 29 par condensation du TEOS et du MTEOS en silice et de générer une porosité pour laquelle la taille moyenne des pores obtenus est inférieure à 2 nm.

Dans le cas des films obtenus par co-condensation de TEOS et MTEOS et afin d'assurer une bonne hydrophobie, une post-méthylation peut être réalisée en plongeant durant 72h l'élément réflecteur 25 dans un mélange d'hexaméthyldisilazane dissout à 20 Vol. % dans du toluène anhydre. Puis l'élément réflecteur 25 est à nouveau traité thermiquement à 350°C pendant 10 min.

Le dispositif de détection 3 d'un taux d'alcoolémie fonctionne par la mesure de l'intensité réfléchie de l'élément réflecteur sensible 25 en fonction de l'intensité incidente émise par la source de lumière 23.

Après calibration, on peut mesurer avec une grande précision, fiabilité et répétabilité le taux d'alcoolémie d'une personne 14 exhalant dans l'enceinte de mesure 11 du dispositif de détection 3 et donc quantifier de manière générale un composé volatil.

Le dispositif de détection 3 se distingue en outre par le fait qu'il possède un encombrement très faible, un temps de réponse rapide et un coût plus faible que les éthylotests électroniques connus à ce jour, tout en permettant une grande fiabilité des mesures. Il permet de plus de détecter de faibles quantités d'éthanol avec précision, avec un seuil bas d'environ 0.02mg/L d'éthanol dans l'air humide.

La sensibilité du dispositif de détection 3 peut en outre être optimisée par un choix judicieux de l'épaisseur de la couche sensible 29 et de la longueur d'onde de la source de lumière 23.

En effet, ci-dessus des équations de Fresnel simplifiées ont été présentées pour expliquer la base de fonctionnement du dispositif de détection 3.

En réalité, ces équations de Fresnel intègrent des termes complexes (c'est-à-dire intègrent des termes comprenant le nombre complexe i avec $i^2=-1$) et tiennent également compte du substrat 27. Ces équations sont résolues de façon numérique par des calculateurs.

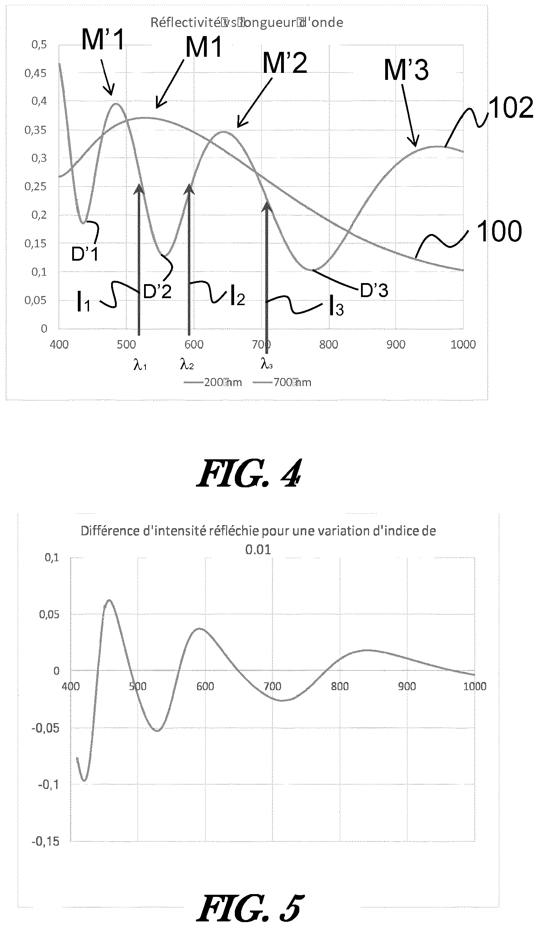

La figure 4 représente un graphe montrant pour deux épaisseurs différentes 200nm (courbe 100) et 700nm (courbe 102) de la couche sensible 29 la réflectivité en fonction de la longueur d'onde $\lambda$ en nm.

Ces courbes ont été obtenues par une résolution numérique des équations de Fresnel complexes tenant compte de la présence du substrat 27.

On constate que la courbe 100 correspondant à la couche sensible 29 de moindre épaisseur ne présente dans le spectre visible qu'un seul pic d'interférence constructive M1 tandis que la courbe 102 correspondant à la couche sensible 29 d'épaisseur plus importante présente trois pics d'interférence constructive M'1, M'2 et M'3 et trois pics d'interférence destructive D'1, D'2 et D'3. La position en longueur d'onde des divers pics peut varier en fonction de l'angle d'incidence $\theta_i$.

Dans le cas de la réflexion d'une couche mince, le fait que les pics d'interférences constructive et destructive soient plus resserrés pour des couches d'épaisseurs plus importantes que pour les couches plus minces est un phénomène connu en soi.

Cependant, de façon judicieuse, on peut déduire de ces spectres de réflectivité une ou plusieurs longueurs d'ondes permettant d'avoir une sensibilité accrue du dispositif de détection 3.

En effet, la figure 5 montre sur un graphe la variation de l'intensité réfléchie en fonction de la longueur d'onde pour une couche d'une épaisseur donnée de 700nm lorsque cette dernière subit une variation d'indice de réfraction d'une valeur prédéfinie de 0,01.

On constate que les maxima de différence d'intensités permettant une analyse optimale avec une grande sensibilité sont obtenus pour les longueurs d'onde $\lambda_1$, $\lambda_2$, $\lambda_3$ correspondantes à une des positions des flèches de la figure 4 qui sont situées au niveau des points d'inflexions entre un pic d'interférence constructive M'1, M'2 ou M'3 et un pic d'interférence destructive D'1, D'2 et D'3, ces points d'inflexions étant signalés par exemple $I_1$, $I_2$ et $I_3$, dans le spectre de la figure 4.

Dans la pratique, l'épaisseur e est choisie suffisamment grande pour exalter les pentes des zones d'inflexions du spectre de réflexion (comme sur la figure 4), sans que le nombre d'interférences ne dépasse une valeur qui rendrait l'alignement trop délicat.

Il s'avère judicieux de choisir ainsi une épaisseur e permettant d'avoir dans une plage de longueurs d'ondes comprises entre 400 et 800 nm au moins deux et au plus trois ou quatre pics d'interférences constructives.

Par ailleurs, une épaisseur e trop importante de la couche sensible 29 entrainerait en outre un temps d'équilibre trop long ce qui pourrait être considéré comme gênant pour un utilisateur du dispositif de détection 3.

La longueur d'onde de la source de lumière 23 étant monochromatique ou quasi-monochromatique et fixe, l'angle d'incidence est alors ajusté de façon à ce que la position en longueur d'onde d'une inflexion du spectre coïncide avec la longueur d'onde de la source de lumière 23.

On peut déterminer ces longueurs d'onde spécifiques pour une couche sensible d'épaisseur e donnée ayant un indice de réfraction $n_2$ et déposée sur un substrat 27 ayant un indice de réfraction $n_3$ ainsi qu'un angle d'incidence $\theta_i$ en calculant le spectre de réflexion $R(\lambda)$ avec les équations complexes et en déterminant les points d'inflexions, par

exemple $I_1$, $I_2$, ou $I_3$ en déterminant les points pour lesquels la dérivée seconde du spectre est égale à zéro $\left( \dfrac{\partial^2 R}{\partial \lambda^2} = 0 \right)$.

Les points d'inflexions peuvent aussi être déterminés expérimentalement en éclairant par exemple l'élément réflecteur 21 par une source de lumière à spectre continue et parallèle sous un angle d'incidence donné et en mesurant la réflectivité en fonction de la longueur d'onde par exemple avec un spectromètre, puis en déterminant les points pour lesquels la dérivée seconde du spectre mesuré est égale à zéro $\left( \dfrac{\partial^2 R}{\partial \lambda^2} = 0 \right)$.

Le dispositif de détection 3 possède une couche de substrat 27 ayant par exemple un indice de réfraction $n_s$ tel que $1.8 < n_3 < 4$, notamment $2.5 < n_3 < 3.5$, une couche sensible 29 d'épaisseur comprise entre 50nm et 3000nm notamment entre 400nm et 1000nm et présentant un indice de réfraction $n_2$ tel que $1.2 < n_2 < 1.6$, plus particulièrement $1.3 < n_2 < 1.4$ pour une longueur d'onde $\lambda$ de la source de lumière 23 comprise entre 500nm et 1000nm.

La méthode décrite ci-dessus permet d'optimiser la sensibilité d'un dispositif de détection 3 optique basé sur la mesure de la réflectivité d'une couche sensible 3 déposée sur un substrat 27.

Le déposant déclare qu'une protection indépendante peut être recherchée pour un dispositif de détection 3 optique basé sur un principe similaire comprenant une couche sensible 29 à un composé spécifique et un substrat 27 qui peut être adapté à la détection par mesure de la réflectivité, contenant notamment de composés volatils, en particulier des composés organiques volatils, notamment des aldéhydes ou des composés aromatiques.

On comprend donc que la sensibilité du dispositif de détection 3 peut être exaltée en choisissant judicieusement en particulier l'épaisseur de la couche sensible d'une part et la longueur d'onde combinée avec l'angle d'incidence d'autre part.

Sur un principe similaire, à celui de la détection de l'alcool, il est possible de réaliser un dispositif adapté à la détection de composés chimiques autre que l'éthanol. Dans ce cas, la couche sensible 23 doit être adaptée pour être sensible et spécifique au composé chimique autre que l'éthanol, par exemple des composés organiques volatils (COV) notamment des aldéhydes et/ou des composés aromatiques.

Comme évoqué ci-dessus notamment en relation avec la figure 3, dans le cas d'une haleine expirée contenant de l'éthanol, le taux d'humidité est de 100% et on a donc pu observer une corrélation linéaire entre la tension de mesure à la sortie du détecteur de lumière 25 et la concentration d'éthanol contenue dans l'air humide.

Cependant, dans certains cas plus généraux, il se peut que le taux d'humidité diffère de 100% de sorte qu'une correction tenant compte de l'influence de l'humidité semble requise pour préserver une bonne précision de mesure. De plus, on a constaté que la température extérieure peut également influer sur les résultats de mesure ainsi que des dérives correspondant par exemple au vieillissement naturel de la source de lumière 23 ou du détecteur 25.

Dans ce cas, il est proposé un nouveau mode de réalisation avec un autre traitement alternatif des signaux de mesure.

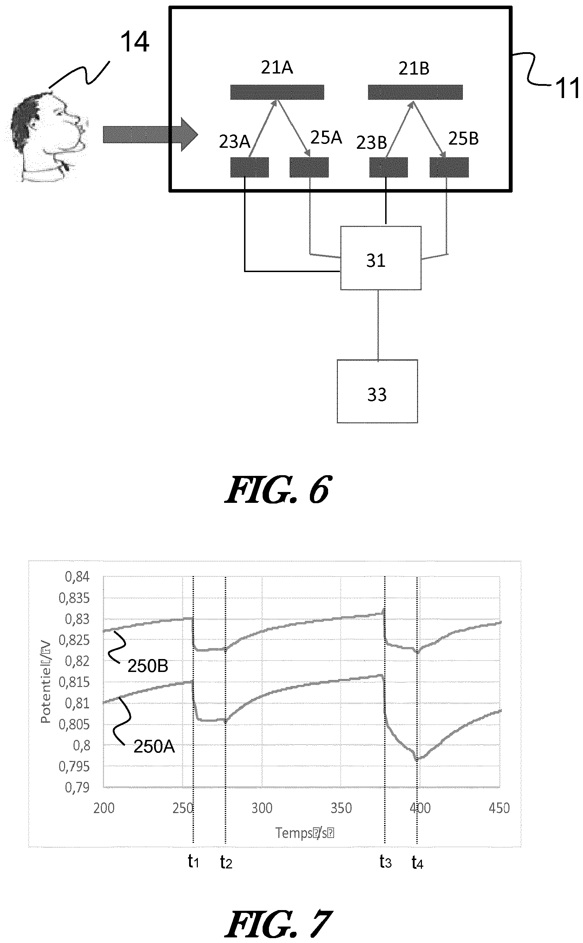

Sur la figure 6 est représentée une enceinte de mesure 11 dans lequel, de façon similaire à la figure 1, une personne 14 souffle en exhalant son haleine. Bien entendu, de façon plus large, n'importe quel flux d'un composé volatil, notamment composé organique volatil COV, pour autant que la couche soit sensible au composé volatil à détecter, peut être introduit dans l'enceinte de mesure 11.

La source de lumière 23A correspond à la source de lumière 23 de la figure 1. Le détecteur de lumière 25A correspond au détecteur de lumière 25 de la figure 1 et l'élément réflecteur sensible 21A correspond au réflecteur sensible 21 de la figure 1.

Dans ce mode de réalisation, le dispositif de détection optique 3 comprend de plus un élément réflecteur sensible additionnel 21B. Ce réflecteur 21B est réalisé de la même manière et selon le même protocole que le réflecteur 21A à la différence près que l'on utilise 100% de précurseur TEOS et 0% de précurseur MTEOS.

L'élément réflecteur sensible additionnel 21B est éclairé par une source de lumière additionnelle 23B et la lumière réfléchie est détectée par le détecteur de lumière additionnel 25B. Le montage entre la source de lumière additionnelle 23B, l'élément réflecteur 23B additionnel et le détecteur de lumière additionnel 25B est en tout point similaire au montage de la source de lumière 23A, l'élément réflecteur 23A et le détecteur de lumière 25A et donc au montage comme expliqué au sujet de la figure 1, seuls les angles d'incidence et de détection peuvent être différents.

Selon une variante, on n'utilise qu'une seule source de lumière et on dispose les éléments réflecteurs côte-à-côte pour être éclairés par la même source de lumière.

Dans ce cas, comme discuté ci-dessus, la couche sensible 29 du réflecteur 21A possède un caractère hydrophobe tandis que la couche sensible du réflecteur 21B, réalisée à partir d'une solution sol-gel 100% TEOS est hydrophile. Cela a pour conséquence que le réflecteur 21B n'absorbe pas (ou pratiquement pas) d'éthanol et que la variation de son indice de réfraction est seulement fonction de l'humidité dans l'atmosphère à tester. Ainsi, le signal de mesure à la sortie du détecteur de lumière 25B varie en fonction du taux d'humidité seulement.

La figure 7 montre sur un graphe en fonction du temps un signal de mesure 250A, par exemple une tension, correspondant à l'intensité de la lumière réfléchie par l'élément réflecteur 21A et un signal de mesure 250B, par exemple une tension, correspondant à l'intensité de la lumière réfléchie par l'élément réflecteur 21A.

A un instant $t_1$, pendant une durée de 20s jusqu'à l'instant $t_2$, on a injecté de la vapeur d'eau dans l'enceinte 11. On voit que l'intensité réfléchie diminue de façon abrupte pour les deux détecteurs de lumière 25A et 25B. Puis, après l'arrêt de l'injection, les signaux de mesure 250A et 250B augmentent, ce qui correspond à un rééquilibrage avec l'atmosphère environnante.

A un instant $t_3$, pendant une durée de 20s jusqu'à un instant $t_4$, on a injecté un mélange de vapeur d'eau et de 0,92mg/L d'éthanol dans l'enceinte 11. On voit que l'intensité réfléchie diminue de façon abrupte pour les deux détecteurs de lumière 25A et 25B, mais de façon plus prononcée pour le détecteur de lumière 25A qui mesure la lumière réfléchie depuis l'élément réfléchissant 21A. Puis, après l'arrêt de l'injection, les signaux de mesure 250A et 250B augmentent, ce qui correspond à un rééquilibrage avec l'atmosphère environnante.

Pour obtenir la teneur en éthanol, il est nécessaire de traiter les signaux de mesures de la façon suivante : Soit $U_{25A}(t)$ l'évolution du signal de mesure dans le temps du détecteur de lumière 25A (ceci correspond donc à la courbe 250A) et soit $U_{25B}(t)$ l'évolution du signal de mesure dans le temps du détecteur de lumière 25B (ceci correspond donc à la courbe 250B).

Dans ce cas, on détermine la différence de la dérivée temporelle des deux intensités mesurées :

$$f(t) = \frac{dU_{25A}}{dt} - \frac{dU_{25B}}{dt}$$

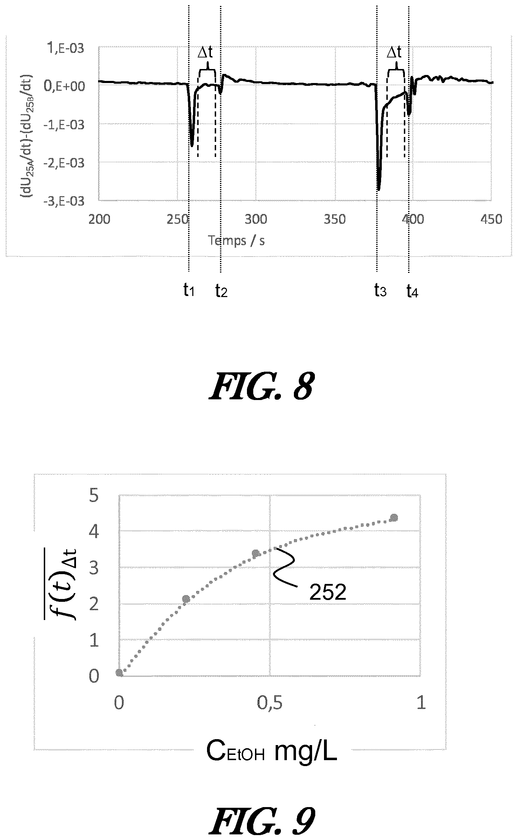

Le résultat de cette opération est montré sur la figure 8.

En cas d'absence d'éthanol entre $t_1$ et $t_2$, on observe un plateau d'une durée $\Delta t$ que l'on appelle aussi zone temporelle d'intérêt prédéterminée pour laquelle la dérivée dans le temps est la même pour les deux intensités mesurées.

$$\Delta t = intervalle\ de\ temps\ avec\ \frac{dU_{25A}}{dt} - \frac{dU_{25B}}{dt} = 0\ en\ absence\ d'éthanol$$

Pour déterminer la teneur en éthanol entre $t_3$ et $t_4$, on va déterminer pendant la zone temporelle d'intérêt prédéterminée la moyenne de f(t) :

$$\overline{f(t)_{\Delta t}} = \int_{\Delta t} f(t)\,dt$$

Cette moyenne $\overline{f(t)_{\Delta t}}$ peut être corrélée via une courbe de corrélation 252 montrée à la figure 9 pour déterminer la teneur en éthanol de l'atmosphère à tester. Cette courbe de calibrage 252 peut être déterminée de façon expérimentale, puis être stockée ou enregistrée sous forme numérique dans l'unité 31 de traitement et de calcul.

Ce mode de réalisation peut être considéré comme plus robuste, car il permet non seulement des corrections pour tenir compte de l'influence de l'humidité, mais aussi tenir compte des dérives associés à la température extérieure, aux conditions de mise en présence de l'air expirée et au vieillissement naturel des divers composants formant le dispositif de détection optique 3.

## Revendications

1. Dispositif optique de détection et de quantification de composés volatils, comportant :

   - un élément réflecteur (21) sensible dont le taux de réflexion varie en fonction du taux d'éthanol contenu dans une atmosphère à tester, l'élément réflecteur (21) sensible comportant:

     ◦ un substrat (27),
     ◦ une couche sensible (29) comprenant de la silice sol-gel à caractère hydrophobe microporeuse présentant une épaisseur supérieure à 250 nm, notamment comprise entre 400 nm et 1200nm, une taille moyenne des pores inférieure à 2nm et une porosité inférieure à 25%, la couche sensible (29) étant destinée à être mise en présence avec une atmosphère à tester, notamment chargée ou non d'éthanol, et plus particulièrement de l'haleine expirée d'une personne (14),

   - une source de lumière (23) monochromatique ou quasi-monochromatique disposée pour éclairer la couche sensible (29) sous un angle incident,
   - un détecteur de lumière (25) pour mesurer l'intensité réfléchie par l'élément réflecteur (21) sous un angle de détection,
   et
   - une unité (31) de traitement et de calcul configurée pour déduire de l'intensité réfléchie par l'élément réflecteur (11) un paramètre correspondant à un taux d'alcoolémie.

**2.** Dispositif de détection selon la revendication 1, **caractérisé en ce que** la couche sensible (29) ne comprend pas d'agents structurants, notamment de CTAB, de DTAB ou de F127.

**3.** Dispositif de détection selon l'une quelconque des revendications 1 à 2, **caractérisé en ce que** le substrat (27) possède un indice de réfraction supérieur à 2.5, notamment supérieur à 3 pour une longueur d'onde comprise entre 250nm et 1500nm.

**4.** Dispositif de détection selon l'une quelconque des revendications 1 à 3, **caractérisé en ce que** le substrat (27) est réalisé en un matériau semi-conducteur, notamment en silicium.

**5.** Dispositif de détection selon l'une quelconque des revendications 1 à 4, **caractérisé en ce que** l'angle incident et l'angle de détection sont respectivement compris entre 30° et 75°.

**6.** Dispositif de détection selon l'une quelconque des revendications 1 à 5, **caractérisé en ce que** la longueur d'onde de la source de lumière (23) est monochromatique et choisie avec l'angle d'incidence de façon à coïncider avec la position en longueur d'onde d'une inflexion ($I_1$, $I_2$, $I_3$) entre deux pics d'interférence constructive et destructive du spectre de réflexion de l'élément réflecteur (21).

**7.** Dispositif de détection selon la revendication 6, **caractérisé en ce que** la longueur d'onde de la source de lumière (23) est comprise entre 500nm et 1000nm.

**8.** Dispositif de détection selon l'une quelconque des revendications précédentes, **caractérisé en ce que** la couche sensible (29) présente un indice de réfraction compris entre 1,2 et 1,6, plus particulièrement compris entre 1,3 et 1,4 pour une longueur d'onde comprise entre 500nm et 1000nm.

**9.** Dispositif de détection selon l'une quelconque des revendications 1 à 8, **caractérisé en ce qu'**il comporte en outre

- un élément réflecteur (21B) sensible additionnel dont le taux de réflexion varie en fonction du taux d'humidité contenu dans une atmosphère à tester,

- un détecteur de lumière (25B) additionnel pour mesurer l'intensité réfléchie par l'élément réflecteur (21B) sensible additionnel sous un angle de détection,
- l'unité (31) de traitement et de calcul étant configurée pour déduire des intensités réfléchies d'une part par l'élément réflecteur (11) et d'autre part par l'élément réflecteur sensible additionnel (21B) un paramètre correspondant à un taux d'alcoolémie en tenant compte de l'influence de l'humidité dans l'atmosphère à tester.

**10.** Procédé de fabrication d'un dispositif de détection optique (3) de détection et de quantification de composés volatils selon l'une quelconque des revendications 1 à 9, **caractérisé en ce que**

- on dépose une couche sensible (29) sur un substrat (27) pour former un élément réflecteur sensible (21),
- on détermine le spectre de réflexion de l'élément réflecteur sensible (21),
- la longueur d'onde de la source de lumière (23) est monochromatique, ou quasi monochromatique, et choisie avec l'angle d'incidence du rayon lumineux sur l'élément réflecteur (21) de façon à coïncider avec la position en longueur d'onde d'une inflexion ($I_1$, $I_2$, $I_3$) du spectre de réflexion de l'élément réflecteur (21).

**11.** Procédé de fabrication selon la revendication 10, **caractérisé en ce que** le substrat (27) possède un indice de réfraction compris entre 1.8 et 4, notamment entre 2.5 et 3.5, la couche sensible (29) possède une épaisseur supérieure à 250 nm, notamment comprise entre 400 nm et 1200nm, et présent un indice de réfraction compris entre 1.2 et 1.6, plus particulièrement entre 1.3 et 1.4 et la longueur d'onde de la source de lumière (23) est comprise entre 500nm et 1000nm.

**12.** Procédé selon les revendications 10 ou 11, **caractérisé en ce que** l'élément réflecteur (21) est réalisé selon le procédé selon la revendication 10 ou 11.

**13.** Procédé de détection optique de détection et de quantification de composés volatils dans une atmosphère à tester, **caractérisé en ce qu'**il comprend les étapes suivantes :

- on mesure l'intensité réfléchie par un élément réflecteur (21A) sensible selon l'une quelconque des revendications 1 à 8 dont le taux de réflexion varie en fonction du taux d'éthanol contenu dans l'atmosphère à tester,
- on mesure l'intensité réfléchie par un élément réflecteur (21B) sensible additionnel selon la revendication 9 dont le taux de réflexion varie en fonction du taux d'humidité contenu dans l'atmosphère à tester,
- dans une zone temporelle prédéterminée d'intérêt (Δt) pour laquelle la dérivée dans le temps est la même pour les deux intensités mesurées en cas d'absence d'éthanol, on détermine la différence de la dérivée temporelle des deux intensités mesurées pour réaliser une corrélation avec une courbe de calibrage (252) afin de quantifier l'éthanol contenu dans l'atmosphère à tester.

14. Procédé de détection optique de détection et de quantification de composés volatils dans une atmosphère à tester selon la revendication 13, **caractérisé en ce que** la longueur d'onde de la source de lumière (23) est choisie de façon à coïncider avec la position en longueur d'onde d'une inflexion ($I_1$, $I_2$, $I_3$) entre deux pics d'interférence constructive et destructive du spectre de réflexion de l'élément réflecteur (21A) sensible.

## Patentansprüche

1. Optische Vorrichtung zum Nachweis und zur Quantifizierung von flüchtigen Verbindungen, die Folgendes umfasst:

   - ein sensitives Reflektorelement (21), dessen Reflexionsgrad in Abhängigkeit vom Ethanolgehalt variiert, der in einer zu prüfenden Atmosphäre enthalten ist, wobei das sensitive Reflektorelement (21) Folgendes umfasst:

     ◦ ein Substrat (27),
     ◦ eine sensitive Schicht (29), die Sol-Gel-Siliciumdioxid mit einer mikroporösen hydrophoben Beschaffenheit umfasst, die eine Dicke von mehr als 250 nm, insbesondere zwischen 400 nm und 1200 nm, eine mittlere Porengröße von weniger als 2 nm und eine Porosität von weniger als 25 % aufweist, wobei die sensitive Schicht (29) dazu vorgesehen ist, mit einer zu prüfenden Atmosphäre, die insbesondere mit Ethanol beladen ist oder nicht, und insbesondere mit dem von einer Person (14) ausgeatmeten Atem in Kontakt gebracht zu werden,

   - eine monochromatische oder quasi monochromatische Lichtquelle (23), die so angeordnet ist, dass die sensitive Schicht (29) unter einem Einfallswinkel bestrahlt wird,
   - einen Lichtdetektor (25) zum Messen der vom Reflektorelement (21) unter einem Erfassungswinkel reflektierten Intensität
   und
   - eine Verarbeitungs- und Recheneinheit (31), die konfiguriert ist, um aus der vom Reflektorelement (11) reflektierten Intensität einen Parameter abzuleiten, der einem Blutalkoholspiegel entspricht.

2. Nachweisvorrichtung nach Anspruch 1, **dadurch gekennzeichnet, dass** die sensitive Schicht (29) Strukturierungsmittel, insbesondere CTAB, DTAB oder F127, nicht umfasst.

3. Nachweisvorrichtung nach einem der Ansprüche 1 bis 2, **dadurch gekennzeichnet, dass** das Substrat (27) einen Brechungsindex von mehr als 2,5, insbesondere mehr als 3, für eine Wellenlänge zwischen 250 nm und 1500 nm aufweist.

4. Nachweisvorrichtung nach einem der Ansprüche 1 bis 3, **dadurch gekennzeichnet, dass** das Substrat (27) aus einem Halbleitermaterial, insbesondere aus Silicium, besteht.

5. Nachweisvorrichtung nach einem der Ansprüche 1 bis 4, **dadurch gekennzeichnet, dass** der Einfallswinkel und der Erfassungswinkel jeweils zwischen 30° und 75° betragen.

6. Nachweisvorrichtung nach einem der Ansprüche 1 bis 5, **dadurch gekennzeichnet, dass** die Wellenlänge der Lichtquelle (23) monochromatisch ist und mit dem Einfallswinkel so ausgewählt ist, dass sie mit der Position in Bezug auf die Wellenlänge eines Wendepunkts ($I_1$, $I_2$, $I_3$) zwischen zwei Peaks einer konstruktiven und destruktiven Interferenz des Reflexionsspektrums des Reflektorelements (21) zusammenfällt.

7. Nachweisvorrichtung nach Anspruch 6, **dadurch gekennzeichnet, dass** die Wellenlänge der Lichtquelle (23) zwischen 500 nm und 1000 nm liegt.

8. Nachweisvorrichtung nach einem der vorhergehenden Ansprüche, **dadurch gekennzeichnet, dass** die sensitive Schicht (29) einen Brechungsindex zwischen 1,2 und 1,6, insbesondere zwischen 1,3 und 1,4, für eine Wellenlänge zwischen 500 nm und 1000 nm aufweist.

9. Nachweisvorrichtung nach einem der Ansprüche 1 bis 8, **dadurch gekennzeichnet, dass** sie außerdem Folgendes umfasst:

   - ein zusätzliches sensitives Reflektorelement (21B), dessen Reflexionsgrad in Abhängigkeit vom Feuchtigkeitsgehalt variiert, der in einer zu prüfenden Atmosphäre enthalten ist,
   - einen zusätzlichen Lichtdetektor (25B) zum Messen der vom zusätzlichen sensitiven Reflektorelement (21B) unter einem Erfassungswinkel reflektierten Intensität,
   - eine Verarbeitungs- und Recheneinheit (31), die konfiguriert ist, um aus Intensitäten, die einerseits vom Reflektorelement (11) und andererseits vom zusätzlichen sensitiven Reflektorelement (21B) reflektiert werden, einen Parameter abzuleiten, der einem Blutalkoholspiegel entspricht, wobei der Einfluss der Feuchtigkeit in der zu prüfenden Atmosphäre berücksichtigt wird.

10. Verfahren zur Herstellung einer optischen Nachweisvorrichtung (3) zum Nachweis und zur Quantifizierung von flüchtigen Verbindungen nach einem der Ansprüche 1 bis 9, **dadurch gekennzeichnet, dass**

   - eine sensitive Schicht (29) auf einem Substrat (27) abgeschieden wird, um ein sensitives Reflektorelement (21) zu bilden,
   - das Reflexionsspektrum des sensitiven Reflektorelements (21) bestimmt wird,
   - die Wellenlänge der Lichtquelle (23) monochromatisch oder quasimonochromatisch ist und mit dem Einfallswinkel des Lichtstrahls auf das Reflektorelement (21) so ausgewählt wird, dass sie mit der Position in Bezug auf die Wellenlänge eines Wendepunkts ($I_1$, $I_2$, $I_3$) des Reflexionsspektrums des Reflektorelements (21) zusammenfällt.

11. Herstellungsverfahren nach Anspruch 10, **dadurch gekennzeichnet, dass** das Substrat (27) einen Brechungsindex zwischen 1,8 und 4, insbesondere zwischen 2,5 und 3,5, aufweist, die sensitive Schicht (29) eine Dicke von mehr als 250 nm, insbesondere zwischen 400 nm und 1200 nm, aufweist, und einen Brechungsindex zwischen 1,2 und 1,6, insbesondere zwischen 1,3 und 1,4, aufweist, und die Wellenlänge der Lichtquelle (23) zwischen 500 nm und 1000 nm liegt.

12. Verfahren nach den Ansprüchen 10 oder 11, **dadurch gekennzeichnet, dass** das Reflektorelement (21) gemäß dem Verfahren nach Anspruch 10 oder 11 hergestellt wird.

13. Optisches Nachweisverfahren zum Nachweis und zur Quantifizierung von flüchtigen Verbindungen in einer zu prüfenden Atmosphäre, **dadurch gekennzeichnet, dass** es die folgenden Schritte umfasst:

   - das Messen der Intensität, die von einem sensitiven Reflektorelement (21A) nach einem der Ansprüche 1 bis 8 reflektiert wird, wobei der Reflexionsgrad in Abhängigkeit vom Ethanolgehalt variiert, der in der zu prüfenden Atmosphäre enthalten ist,
   - das Messen der Intensität, die von einem zusätzlichen sensitiven Reflektorelement (21B) nach Anspruch 9 reflektiert wird, wobei der Reflexionsgrad in Abhängigkeit vom Feuchtigkeitsgehalt variiert, der in der zu prüfenden Atmosphäre enthalten ist,
   - in einem interessierenden vorbestimmten Zeitbereich ($\Delta t$), für den im Fall des Fehlens von Ethanol die zeitliche Ableitung für die beiden gemessenen Intensitäten gleich ist, das Bestimmen der Differenz der zeitlichen Ableitung der gemessenen beiden Intensitäten, um eine Korrelation mit einer Kalibrierungskurve (252) durchzuführen, um das Ethanol zu quantifizieren, das in der zu prüfenden Atmosphäre enthalten ist.

14. Optisches Nachweisverfahren zum Nachweis und zur Quantifizierung von flüchtigen Verbindungen in einer zu prüfenden Atmosphäre nach Anspruch 13, **dadurch gekennzeichnet, dass** die Wellenlänge der Lichtquelle (23) so ausgewählt ist, dass sie mit der Position in Bezug auf die Wellenlänge eines Wendepunkts ($I_1$, $I_2$, $I_3$) zwischen zwei Peaks einer konstruktiven und destruktiven Interferenz des Reflexionsspektrums des sensitiven Reflektorelements (21A) zusammenfällt.

**Claims**

1.  Optical device for detecting and quantifying volatile compounds, comprising:

    - a sensitive reflective element (21) with a reflection rate which varies as a function of the ethanol content contained in an atmosphere to be tested, the sensitive reflective element (21) comprising:

        ∘ a substrate (27),
        ∘ a sensitive layer (29) comprising microporous hydrophobic sol-gel silica having a thickness of greater than 250 nm, notably of between 400 nm and 1200 nm, a mean pore size of less than 2 nm and a porosity of less than 25%, the sensitive layer (29) being intended to be placed in the presence of an atmosphere to be tested, notably which may or may not be charged with ethanol, and more particularly the breath exhaled from an individual (14),

    - a monochromatic or quasi-monochromatic light source (23) arranged to illuminate the sensitive layer (29) under an incident angle,
    - a light detector (25) for measuring the intensity reflected by the reflective element (21) under an angle of detection,
    and
    - a processing and calculation unit (31) configured to deduce from the intensity reflected by the reflective element (11) a parameter corresponding to a blood alcohol content.

2.  Detection device according to Claim 1, **characterized in that** the sensitive layer (29) does not comprise structuring agents, notably CTAB, DTAB or F127.

3.  Detection device according to either one of Claims 1 and 2, **characterized in that** the substrate (27) has a refractive index greater than 2.5, notably greater than 3 for a wavelength of between 250 nm and 1500 nm.

4.  Detection device according to any one of Claims 1 to 3, **characterized in that** the substrate (27) is made of a semiconductive material, notably of silicon.

5.  Detection device according to any one of Claims 1 to 4, **characterized in that** the incident angle and the angle of detection are respectively between 30° and 75°.

6.  Detection device according to any one of Claims 1 to 5, **characterized in that** the wavelength of the light source (23) is monochromatic and chosen with the angle of incidence so as to coincide with the position in terms of wavelength of an inflection ($I_1$, $I_2$, $I_3$) between two constructive and destructive interference peaks of the reflection spectrum of the reflective element (21).

7.  Detection device according to Claim 6, **characterized in that** the wavelength of the light source (23) is between 500 nm and 1000 nm.

8.  Detection device according to any one of the preceding claims, **characterized in that** the sensitive layer (29) has a refractive index of between 1.2 and 1.6, more particularly between 1.3 and 1.4 for a wavelength of between 500 nm and 1000 nm.

9.  Detection device according to any one of Claims 1 to 8, **characterized in that** it also comprises

    - an additional sensitive reflective element (21B) with a reflection rate which varies as a function of the moisture content contained in an atmosphere to be tested,
    - an additional light detector (25B) for measuring the intensity reflected by the additional sensitive reflective element (21B) under an angle of detection,
    - the processing and calculation unit (31) being configured to deduce from the intensities reflected, on the one hand, by the reflective element (11) and, on the other hand, by the additional sensitive reflective element (21B) a parameter corresponding to a blood alcohol content while taking into account the influence of the moisture in the atmosphere to be tested.

10. Method for producing an optical detection device (3) for detecting and quantifying volatile compounds according to any one of Claims 1 to 9, **characterized in that**

- a sensitive layer (29) is deposited on a substrate (27) in order to form a sensitive reflective element (21),
- the reflection spectrum of the sensitive reflective element (21) is determined,
- the wavelength of the light source (23) is monochromatic, or quasi-monochromatic, and chosen with the angle of incidence of the light rayon the reflective element (21) so as to coincide with the position in terms of wavelength of an inflection ($I_1$, $I_2$, $I_3$) of the reflection spectrum of the reflective element (21).

11. Production method according to Claim 10, **characterized in that** the substrate (27) has a refractive index of between 1.8 and 4, notably between 2.5 and 3.5, the sensitive layer (29) has a thickness of greater than 250 nm, notably of between 400 nm and 1200 nm, and has a refractive index of between 1.2 and 1.6, more particularly between 1.3 and 1.4, and the wavelength of the light source (23) is between 500 nm and 1000 nm.

12. Method according to Claim 10 or 11, **characterized in that** the reflective element (21) is produced according to the method according to Claim 10 or 11.

13. Optical detection method for detecting and quantifying volatile compounds in an atmosphere to be tested, **characterized in that** it comprises the following steps:

- the intensity reflected by a sensitive reflective element (21A) according to any one of Claims 1 to 8, with a reflection rate which varies as a function of the ethanol content contained in the atmosphere to be tested, is measured,
- the intensity reflected by an additional sensitive reflective element (21B) according to Claim 9, with a reflection rate which varies as a function of the moisture content contained in the atmosphere to be tested, is measured,
- in a predetermined time zone of interest ($\Delta t$) for which the time derivative is the same for the two intensities measured in the case of the absence of ethanol, the difference in the time derivative of the two intensities measured is determined in order to carry out a correlation with a calibration curve (252) in order to quantify the ethanol contained in the atmosphere to be tested.

14. Optical detection method for detecting and quantifying volatile compounds in an atmosphere to be tested according to Claim 13, **characterized in that** the wavelength of the light source (23) is chosen so as to coincide with the position in terms of wavelength of an inflection ($I_1$, $I_2$, $I_3$) between two constructive and destructive interference peaks of the reflection spectrum of the sensitive reflective element (21A).

*FIG. 1*

*FIG. 2A*

FIG. 2B

$y = -1,2786x + 6,4295$
$R^2 = 0,9976$

Potentiel mesuré (V)

Concentration d'éthanol contenu dans l'air humide (mg/l)

FIG. 3

## FIG. 4

## FIG. 5

**FIG. 6**

**FIG. 7**

## FIG. 8

## FIG. 9

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Porosity and mechanical properties of mesoporous thin films assessed by environmental ellipsometric porosimetry. **CEDRIC BOISSIÈRE et al.** Langmuir : the ACSjournal of surfaces and colloids. American Chemical Society, 2005, vol. 21, 12362-71